**Europäisches Patentamt**

**European Patent Office**

**Office européen des brevets**

(19)

(11) Veröffentlichungsnummer: **0 021 135**
**B1**

(12)                    **EUROPÄISCHE  PATENTSCHRIFT**

(45) Veröffentlichungstag der Patentschrift:
25.05.83

(51) Int. Cl.³: **A 61 K  7/02,** A 61 K  7/48

(21) Anmeldenummer: 80103065.1

(22) Anmeldetag: 03.06.80

(54) Verfahren zur Herstellung eines Kosmetikstifts.

(30) Priorität: 07.06.79  DE 2923080

(43) Veröffentlichungstag der Anmeldung:
07.01.81 Patentblatt 81/1

(45) Bekanntmachung des Hinweises auf die Patenterteilung:
25.05.83 Patentblatt 83/21

(84) Benannte Vertragsstaaten:
CH FR GB IT LI

(56) Entgegenhaltungen:
DE-A-2 646 434
DE-B-2 015 045
DE-C-1 000 713
GB-A-1 442 426
US-A-2 101 843
PATENTS ABSTRACTS OF JAPAN, Band 3, Nr. 3
(C-33), 16. Januar 1979, Seite 158 C 33, Tokyo, JP.
PATENTS ABSTRACTS OF JAPAN, Band 3, Nr. 75
(C-50), 27. Juni 1979, Seite 33 C 50, Tokyo, JP.
JAPANESE PATENTS GAZETTE, Woche A49,
24. Januar  1979,  Seite 6,  Zusammenfassung
Nr. 88621, Derwent, London, GB.

(73) Patentinhaber: **Schwan-STABILO Schwanhäusser
GmbH & Co., Maxfeldstrasse 3, D-8500 Nürnberg (DE)**

(72) Erfinder: **Hempel Matthias, Dr., Haagstrasse 35,
D-8501 Eckental 1 (DE)**
Erfinder: **Brüchert, Werner, Blücher Strasse 42,
D-8500 Nürnberg (DE)**

(74) Vertreter: **LOUIS, PÖHLAU, LOHRENTZ & SEGETH;
Kesslerplatz 1, D-8500 Nürnberg (DE)**

Die Akte enthält technische Angaben, die nach dem
Eingang der Anmeldung eingereicht wurden und die
nicht in dieser Patentschrift enthalten sind.

**0 021 135**

## Verfahren zur Herstellung eines Kosmetikstiftes

Die Erfindung bezieht sich auf ein Verfahren zur Herstellung eines Fettstoffe salbenförmiger oder fester Konsistenz und gegebenenfalls Füllstoffe enthaltenden Kosmetikstifts zur Erzeugung eines cremigen Auftrags auf angefeuchtete Haut.

Ein für die bekannten Cremes charakteristisches Merkmal ist ein verhältnismäßig großer Wasseranteil. Durch die Einarbeitung von Emulgatoren kann dieser Anteil bis zu 70 Gew.-% betragen. Hierdurch erhalten die Cremes ihre typische (crem- oder salbenartige) Konsistenz, die nicht nur für ihre Abfüllung in Tuben oder Dosen, sondern auch für ihre Handhabung beim Aufbringen auf die Haut Bedeutung hat. Dabei geht die Verbrauchergewohnheit dahin, daß die Creme dem Vorratsbehälter mit den Fingern entnommen und dann auf die zu behandelnden Körperstellen aufgebracht wird. Bei einer solchen Cremeentnahme kommt zwangsläufig auch der in dem Behälter zurückbleibende, nicht verbrauchte Cremeinhalt mit den Fingern in solchen Kontakt, daß eine hohe Wahrscheinlichkeit für einen Befall des Inhaltes mit für die Gesundheit und/oder Lagerbeständigkeit schädlichen Mikroorganismen besteht. Die Voraussetzungen für eine derartige Kontamination der Creme sind wegen ihres hohen Feuchtigkeitsgehaltes besonders günstig.

Die Erfindung will zu einer Lösung des sich herausergebenen hygienischen Problems beitragen. Dies soll u. a. dadurch erreicht werden, daß die Creme wie mit einem Stift auf die Haut aufgebracht werden kann.

Kosmetische Stifte, vermittels derer sich ein cremiger Abstrich auf der Haut erzeugen läßt, sind allerdings schon in vielfältiger Ausführung bekannt, so z. B. als Lippen- und Deodorant-Stifte. In den bekannten Fällen besitzen die Kosmetikstifte eine vergleichsweise weiche Konsistenz, was durch den Feuchtigkeitsgehalt und/oder die Art des verwendeten Bindemittels bedingt ist. Solche Stifte sind wie die bekannten Cremes der Gefahr der Kontamination ausgesetzt. In hygienischer Hinsicht besteht dabei der Nachteil, daß sich wegen der verhältnismäßig geringen Festigkeit der Stifte nach deren Gebrauch keine »frische« Auftragsfläche durch Anspitzen des Stiftauftragsendes erzeugen läßt.

Soweit die bekannten Kosmetikstifte im Gießverfahren hergestellt werden, ergibt sich der weitere Mangel, daß die Einarbeitung größerer Anteile an Feststoffen nicht möglich ist. Im Hinblick auf spezielle Anwendungszwecke ist jedoch vielfach ein hoher Feststoffgehalt erwünscht.

Hiernach ist die Aufgabe der Erfindung darin zu sehen, einen Kosmetikstift zur Erzeugung eines cremigen Auftrages zur Verfügung zu stellen, der im Vergleich zu den bekannten Cremes und Kosmetikstiften in sehr viel geringerem Maße der Kontamination ausgesetzt ist und eine hinreichende Festigkeit besitzt, um zur Freilegung einer frischen Auftragsfläche angespitzt werden zu können. Im Hinblick auf die an sich gegensätzlichen Anforderungen, die in der Vorgabe enthalten sind, eine Creme mit hoher Festigkeit bereitzustellen, soll der Stift so beschaffen sein, daß er im Gegensatz zu den bekannten Stiftausführungen nur auf feuchter Haut einen cremigen Auftrag hinterläßt.

Das erfindungsgemäße Verfahren zur Herstellung eines solchen Stiftes besteht darin, daß unter Zugabe von Wasser aus den salbenförmigen oder festen Fettstoffen und gegebenenfalls den Füllstoffen zusammen mit wasserlöslichen, die Feststoffe nach dem Wasserentzug verklebenden Bindemitteln und die Fettstoffe auf der befeuchteten Haut emulgierenden Substanzen eine kalt verformbare Masse bereitet und dieser nach der Kaltverformung das Wasser so weit entzogen wird, daß der bei der Kaltverformung erhaltene Stift unter Bildung einer festen Struktur zu einem anspitzbarem Körper erhärtet.

Durch die Einarbeitung der genannten Bindemittel erhält der erfindungsgemäße Cremestift einerseits die angestrebte hohe Festigkeit, andererseits aber auch seinen Cremecharakter, weil die Bindemittel die Fettstoffe und gegebenenfalls anwesenden Füllstoffe (erst) unter Einwirkung der auf der Haut befindlichen Feuchtigkeit freigeben. Der cremige Auftrag auf der Haut kommt dabei aufgrund der in der Stiftmasse enthaltenen Emulgatoren im Zusammenwirken mit dem Feuchtigkeitsfilm der Haut zustande.

Wenn im Zusammenhang mit dem erfindungsgemäßen Verfahren davon die Rede ist, daß der Wasserentzug der Formstücke so weit erfolgen soll, daß eine »feste Struktur« erhalten wird, dann soll hierunter eine Struktur verstanden sein, die sich ohne Zerstörung nicht mehr verformen läßt. Der Wasserentzug erfolgt vorzugsweise durch Lufttrocknung. Der Rest-Wassergehalt der Formstücke wird vielfach zwischen 2 und 5 Gew.-% liegen. Wegen der hohen mechanischen Festigkeit kann der erfindungsgemäß erhältliche Cremestift in Holz zu spitzbaren Stiften verleimt oder auch in Kreideform in Hülsen eingebracht werden.

Nach der Erfindung erhält man eine kosmetische Masse, die als Instantcreme angesehen werden kann und sich gegenüber den herkömmlichen Cremes vor allem durch Wasserfreiheit bzw. vergleichsweise sehr geringen Wassergehalt auszeichnet. Die Gefahr einer Kontamination ist bei einer solchen Masse wesentlich herabgesetzt.

Zur Durchführung des erfindungsgemäßen Verfahrens eignen sich für die Cremebasis an Fettstoffen alle in der Kosmetik üblichen Fette, Öle und Wachse, gesättigte und ungesättigte Fettsäuren und deren Ester mit höheren Alkoholen bzw. deren Glyceride sowohl in salbenförmiger als auch fester Konsistenz.

2

Als Emulgatoren können praktisch alle physiologisch einwandfreien Stoffe dieser Art, z. B. Fettsäuresorbitanester, Polyäthylenoxid und dessen zahlreiche Abkömmlinge sowie auch ionogen aufgebaute Emulgatoren Anwendung finden. Den Emulgatoren fällt die Aufgabe zu, die in dem Stift enthaltenen Fettstoffe mit der auf die Haut aufgebrachten Feuchtigkeit zu einem cremigen Auftrag zu emulgieren.

Als Füllstoffe kommen beispielsweise Kaolin, Talkum, Calciumcarbonat, Metallseifen, Glimmer oder Farbstoffe, sowohl in löslicher als auch in Pigmentform in Betracht.

Als Beispiele für die erfindungsgemäß verwendeten wasserlöslichen Bindemittel seien genannt: Polyvinylpyrrolidon, Carboxymethylcellulose, Dextrin, Tragant.

Bei der Durchführung des erfindungsgemäßen Verfahrens wird im allgemeinen so vorgegangen, daß die einzelnen Bestandteile zusammen mit dem Bindemittel und einer für die Auflösung des Bindemittels notwendige Wassermenge — ca. 20 bis 30% der Gesamtmasse — zunächst verknetet werden. Die entstandene knetbare Masse wird dann im Wege der Kaltverformung, vorzugsweise durch Extrudieren, in zylindrische Formstücke gebracht, welchen dann in bekannter Weise (Lufttrocknung ggf. bei Temperaturen oberhalb Raumtemperatur) das Wasser so weit entzogen wird, daß die angestrebte feste Struktur entsteht.

Neben den bereits genannten Füllstoffen können in die Stiftmasse auch noch Wirkstoffe mit kosmetischer und/oder medizinischer Wirkung eingearbeitet werden.

Nachstehend sind einige Ausführungsbeispiele für die Zusammensetzung nach dem erfindungsgemäßen Verfahren verarbeitbarer Massen angegeben, wobei die für die Verformbarkeit der Massen im Wege der Kaltverformung notwendige Menge Wasser noch zuzugeben ist. Nach der Verformung wird der Stiftmasse das Wasser so weit entzogen, bis eine ausreichend feste Struktur entstanden ist.

## 0 021 135

### Beispiel 1

Die nachfolgend angegebene Zusammensetzung ist für eine kosmetische Masse mit hautpflegener Wirkung bestimmt.

| | | | Gew.-Anteile |
|---|---|---|---|
| Farbstoffe: | TiO$_2$ | | 7 |
| | Eisenoxid Farbstoffe | | 2 |
| Emulgatoren: | Kondensationsverbindung aus Cetyl- und Stearylalkohol mit 20 Mol Äthylenoxid | | 5 |
| | Sorbitanstearat | | 1 |
| Cremebasis: | Vaseline | | 3,5 |
| | Arachidylpropionat | | 2,5 |
| | Di-n-Butyladipat | | 2 |
| Wirkstoffe: | Gemisch von Fettsäureestern aus der PCL-Gruppe der Firma DRAGOCO | | 2 |
| | Weizenkeim-Glyceride | | 2,5 |
| | Natriumsalz einer Polyhydroxycarbonsäure | | 1 |
| | Quaternium 23*) | | 0,5 |
| | Glycerin | | 1 |
| Binder: | Polyvinylpyrrolidon | | 5,5 |
| Füllstoffe: | Kaolin | | 25 |
| | Talkum | | 25 |
| | Glimmer | | 14,5 |
| | | | 100 |

*) = Quaternäres Ammoniumpolymer erhalten durch Reaktion von Dimethylsulfat mit einem Mischpolymerisat aus Vinylpyrrolidon und Dimethylaminoäthylmethacrylat.

**0 021 135**

### Beispiel 2

Die nachfolgende Zusammensetzung ist für eine kosmetische Masse mit sowohl hautpflegender als auch desinfizierender Wirkung geeignet.

|  |  | Gew.-Anteile |
| --- | --- | --- |
| Farbstoffe: | Perlpigmente | 4 |
|  | $TiO_2$ | 1,5 |
|  | Eisenoxid Pigmente | 2,5 |
| Emulgatoren: | Gemisch aus langkettigen Alkoholen mit nichtionogenem Emulgator | 5 |
|  | Polysorbat 20 (20 Mol Äthylenoxid) | 1 |
| Cremebasis: | Vaseline | 4 |
|  | Dioctyladipat, Octylstearat, Octylpalmitat | 2,5 |
|  | n-Butylstearat | 0,5 |
| Wirkstoffe: | PCL W/OE*) | 2 |
|  | Weizenkeimglyceride | 2,5 |
|  | Natriumsalz einer Polyhydroxy-Carbonsäure | 1 |
|  | Quaternium 23 | 1 |
|  | Glycerin | 1,5 |
|  | Alkyldimethylbenzylammonium-Saccharinat | 2,5 |
| Binder: | Carboxymethylcellulose | 5 |
| Füllstoffe: | Kaolin | 24,5 |
|  | Talkum | 24,5 |
|  | Glimmer | 14,5 |
|  |  | 100 |

*) = Mischung eines gereinigten Ölsäureesters und einer hochwertigen Lanolin-Alkohol-Fraktion mit 36% eines Gemisches alkylverzweigter und langkettiger Fettsäureester, hergestellt von der Firma DRAGOCO.

Beispiel 3

Die nachfolgende Zusammensetzung ist für eine Masse mit pflegender und heilender Wirkung bestimmt.

| | | | Gew.-Anteile |
|---|---|---|---|
| Farbstoffe: | Perlpigmente | | 8 |
| | Eisenoxidpigmente | | 2,5 |
| Emulgatoren: | Glycerin Mono-Di-Ester der Palmitin/Stearinsäure | | 5 |
| | N-$\beta$-Hydroxyäthyl-N-$\beta$-Carboxyäthyl-Fettsäure Amido Äthyl Amin, Na-Salz | | 1,5 |
| Cremebasis: | Vaseline | | 4 |
| | Arachidylpropionat | | 2,5 |
| | n-Butylstearat | | 0,5 |
| Wirkstoffe: | Sulfosuccinat-Derivat | | 1,5 |
| | Na-Salz einer Polyhydroxy-Carbonsäure | | 1,5 |
| | Quaternium 23 | | 1 |
| | Calendula Öl | | 1 |
| | Allantoin | | 0,5 |
| | Kamillenextrakt | | 0,5 |
| | Biobranil (DRAGOCO) | | 1,5 |
| Binder: | Dextrin | | 5 |
| Füllstoffe: | Kaolin | | 24 |
| | Talkum | | 24 |
| | Glimmer | | 15,5 |
| | | | 100 |

**0 021 135**

**Patentansprüche**

1. Verfahren zur Herstellung eines Fettstoffe salbenförmiger oder fester Konsistenz und gegebenenfalls Füllstoffe enthaltenden Kosmetikstifts zur Erzeugung eines cremigen Auftrags auf angefeuchtete Haut, dadurch gekennzeichnet, daß unter Zugabe von Wasser aus den Fettstoffen und gegebenenfalls den Füllstoffen zusammen mit wasserlöslichen, die Feststoffe nach dem Wasserentzug verklebenden Bindemitteln und die Fettstoffe auf der befeuchteten Haut emulgierenden Substanzen eine kaltverformbare Masse bereitet und dieser nach der Kaltverformung das Wasser soweit entzogen wird, daß der bei der Kaltverformung erhaltene Stift unter Bildung einer festen Struktur zu einem anspitzbaren Körper erhärtet.

2. Verfahren nach Anspruch 1, dadurch gekennzeichnet, daß den Stiftkörpern nach der Kaltverformung das Wasser bis 2—5 Gew.-% entzogen wird.


**Claims**

1. Process for the manufacture of a cosmetic pencil, containing fatty materials of pasty or solid consistency and, if appropriate, fillers, for producing a creamy application on moistened skin, characterized in that a composition which can be shaped in the cold is prepared, with the addition of water, from the fatty materials and, if appropriate, the fillers together with water-soluble binders which bond the solids after the removal of water and with substances which emulsify the fatty materials on the moistened skin and, after the cold shaping, water is removed from this composition to such a degree that the pencil, obtained in the cold shaping, hardens with the formation of a solid structure, to give a body which can be sharpened.

2. Process according to claim 1, characterized in that water is removed down to 2—5% by weight from the pencil bodies after cold shaping.


**Revendications**

1. Procédé de préparation d'un bâton ou crayon cosmétique contenant des matières grasses de la constistance d'une pommade ou solide et éventuellement des charges pour l'obtention d'un dépôt crémeux sur la peau humidifiée, caractérisé en ce que, on prépare, sous addition d'eau, une masse transformable à froid à partir des matières grasses et éventuellement des charges, ensemble avec des liants solubles dans l'eau collant les matières grasses après l'élimination de l'eau et les substances émulsionnant les matières grasses sur la peau humidifiée et en ce que l'on élimine autant d'eau de la masse précitée après la transformation à froid, qu'il est nécessaire pour que le bâton ou le crayon obtenu au cours de la transformation à froid durcisse sous formation d'une structure solide en un corps taillable ou aiguisable.

2. Procédé suivant la revendication 1, caractérisé en ce qu'après la transformation à froid on élimine de 2 à 5% en poids d'eau du corps en forme de bâton ou crayon.